# EUROPÄISCHE PATENTSCHRIFT

(11) **EP 0 234 508 B1**
(45) Veröffentlichungstag und Bekanntmachung des Hinweises auf die Patenterteilung: **13.10.1993**
(21) Anmeldenummer: 87102380.0
(22) Anmeldetag: 19.02.1987
(51) Int. Cl.: B01D 3/34, C07C 41/42, C07C 45/78, C07C 45/82, C07C 29/80

(54) **Verfahren zur kontinuierlichen Abtrennung von Wasser aus Gemischen mit organischen Substanzen**
Process for the continuous separation of water from a mixture with organic substances
Procédé de séparation continue de l'eau d'un mélange avec des substances organiques

(30) Priorität: 26.02.1986 DE 3606121
(43) Veröffentlichungstag der Anmeldung: 02.09.1987
(73) Patentinhaber: RWE-DEA Aktiengesellschaft für Mineraloel und Chemie, 22204 Hamburg (DE)
(72) Erfinder: Osterburg, Günther, D-4137 Rheurdt 2 (DE); Prezelj, Milan, D-6000 Frankfurt/Main 70 (DE)
(74) Vertreter: Schupfner, Gerhard D.

(56) Entgegenhaltungen:
- DE-A- 2 539 737
- DE-A- 2 720 405
- FR-C- 1 399 555
- US-A- 3 031 384

## Beschreibung

Die Erfindung betrifft ein Verfahren zur kontinuierlichen Abtrennung von Wasser aus einem wasserhaltigen Gemisch von Methyl-tert-butylether und C₄-Kohlenwasserstoffen bei der Auftrennung des Gemisches in einer Destillationskolonne.

Es ist bekannt, daß bei der destillativen Auftrennung von Stoffgemischen die Anwesenheit von Wasser erhebliche Trennprobleme mit sich bringt. Dies gilt insbesondere dann, wenn eine der Komponenten des Stoffgemisches für Wasser ein Lösungsvermittler ist.

Solche Lösungsvermittler für Wasser sind in der Regel die im Destillationseinsatz enthaltenen und bei der destillativen Trennung zu reinigenden Produkte. Es sind immer die unter den Destillationsbedingungen am höchsten siedenden Produkte, die demgemäß auch am Sumpf der Destillationskolonne anfallen. Lösungsvermittler für Wasser sind z.B. Methyl-tert-butylether, Methylethyl-keton und sec-Butylalkohol.

Gemäß der DE-OS 25 47 380 wird das Reaktionsprodukt der Methyl-tert-butylether(MTBE)-Synthese, das neben C₄-Kohlenwasserstoffen (C₄-KW) im wesentlichen MTBE und nicht umgesetztes Methanol und in untergeordneten Mengen tert-Butylalkohol (TBA) und DME enthält, zunächst einer Wasserwäsche unterworfen, um Methanol zu entfernen. Dabei wird durch Lösungsvermittlung seitens MTBE Wasser in die methanolfrei gewaschene Raffinatphase des Extraktors überführt. Der Wassergehalt in dieser Raffinatphase ist abhängig von der MTBE-Konzentration, und diese ist wiederum abhängig von der Isobutenkonzentration im Einsatz-C₄-Schnitt der MTBE-Synthese. Bei geringen Isobutenkonzentrationen werden geringe Mengen MTBE gebildet, die damit auch nur wenig Wasser in die Raffinatphase lösen. Dieses Wasser kann mit der relativ großen Menge an inerten C₄-KW leicht bei deren Abtrennung vom MTBE azeotrop ausgetragen und abgetrennt werden. Bei hohen Isobutenkonzentrationen werden aber entsprechend große MTBE-Mengen gebildet, die so viel Wasser in die Raffinatphase lösen, daß dieses gegebenenfalls mit der vorhandenen Menge an inerten C₄-KW bei der Abtrennung von MTBE nicht mehr ohne weiteres ausreichend azeotrop ausgetragen werden kann. Ein ausreichender Wasseraustrag kann dann nur durch eine entsprechend erhöhte Verdampfung von C₄-KW (Rücklauferhöhung) erreicht werden, oder es ist eine nachträgliche Trocknung des erzeugten MTBE erforderlich.

Bei der Herstellung von Methylethylketon (MEK) aus wasserhaltigem sec-Butylalkohol (SBA), sei es durch Dehydrierung oder durch Oxidation, fallen mehr oder weniger wasserhaltige Rohprodukte an. Nach dem in der DE-OS 23 47 097 beschriebenen Verfahren sind z.B. Wassergehalte zwischen 3 bis 15 % zu erwarten. Solche Reaktionsprodukte werden zweckmäßigerweise vor der Isolierung des MEK zunächst getrocknet. Die Trocknung geschieht in der Regel mit Hilfe azeotroper Schleppmittel wie z.B. Benzol, Hexan, Cyclohexan, Heptan.
In der US-PS 3 228 985 ist ein mehrstufiges Verfahren zur Reinigung von MEK beschrieben, in dem der zu reinigende Strom zuerst mit Natriumcarbonatlösung extraktiv destilliert, dann mit Pentan unter Bildung einer wäßrigen Phase behandelt und das Restwasser der organischen Phase azeotrop abdestilliert wird.
Der Energieverbrauch solcher azeotropen Trocknungen wird im wesentlichen von der Zusammensetzung der ternären Azeotrope und deren Phasenzerfall bestimmt.

Bei der konventionellen Herstellung von SBA durch indirekte Hydratisierung mit Hilfe von z.B. Schwefelsäure als Katalysator fällt ein wasserhaltiger Rohalkohol an.

In den Patentschriften GB-PS 829 424, US-PS 2 875 138 (inhaltlich gleich mit der DE-OS 1 017 602) und der DE-OS 2 033 707 werden Destillationsverfahren zur Reinigung von wäßrigem Roh-SBA beschrieben. Bei diesen Verfahren wird das im Rohalkohol enthaltene Wasser gemeinsam mit leichter siedenden Nebenprodukten bzw. Verunreinigungen in einer Trennkolonne abgetrennt. Die Bildung von Azeotropen mit Wasser ist dabei eine grundsätzliche Voraussetzung für die Abtrennbarkeit von Nebenprodukten bzw. Verunreinigungen wie z.B. Di-sec-butylether (DSBE) und C₈-Kohlenwasserstoffen aus SBA. Andererseits kann das im Roh-SBA enthaltene Wasser, das selbst mit SBA ein SBA-reiches homogenes Azeotrop bildet, mit solchen Nebenprodukten aus SBA abgetrennt werden, ohne daß zu große Mengen SBA mit dem Wasser aus dem zu reinigenden SBA ausgetragen werden. Es wird ein trockener SBA erhalten.

Aus der Zusammensetzung des sich einstellenden ternären azeotropen Gemisches und dem sich aus dieser heterogenen ternären Zusammensetzung ergebenden Löslichkeitsprodukt leiten sich daher die folgenden alternativen Bedingungen für die Destillation ab: Entweder muß eine ausreichende Menge Wasser für die Abtrennung der in die Destillation eingebrachten Menge azeotrop leichter siedender Nebenprodukte oder eine ausreichende Menge azeotrop leichter siedender Nebenprodukte für die Abtrennung der in die Destillation eingebrachten Menge Wasser angeboten werden.

So wird z.B. in der GB-PS 829 424 insbesondere die Problematik beschrieben, wie bei der Destillation von wäßrigem Roh-SBA die Abtrennung von Wasser und von azeotrop leichter siedenden Nebenprodukten in einer Kolonne durch geregelte Rückführung von wäßriger Phase und organischer Phase aus dem Kopfproduktabscheider unter Beibehaltung einer stabilen Gleichgewichtslage in der Kolonne erreicht werden kann.

Diese Beispiele verdeutlichen, daß die Wasserabtrennung aus solchen Produktsystemen immer mit einem erheblichen apparativen Aufwand bzw. Energieverbrauch verbunden ist. So bedient man sich in der Regel der Bildung binärer und/oder ternärer azeotroper Gemische und ist gezwungen, mit dem Wasseraustrag angepaßten Rücklaufverhältnissen zu fahren, separate Trocknungskolonnen zu betreiben oder aber im Seitenstrom der Kolonne, wie es z.B. die DE-PS 24 07 949 vorsieht, mit einem Molsieb Trocknungen vorzunehmen.

Andererseits hat man versucht, bei organischen Substanzen, die zumindest teilweise in jedem Verhältnis mit Wasser mischbar sind, eine Wasserabtrennung durch die Verwendung von Cyclohexan als Schleppanteil zu erreichen (FR-C-1 399 555). Für solche organischen Substanzen, die allerdings nur sehr begrenzt Wasser aufnehmen vermögen, ist dieses Verfahren nicht geeignet.

Insodern bietet auch US-A-3 031 384 keine zufriedenstellende Lösung, weil die Verwendung von Aceton aufgrund der Mischbarkeit mit Wasser gerade nicht zu einer Wasserabscheidung führt.

Es bestand daher die Aufgabe, ein einfach zu handhabendes Verfahren zu entwickeln, bei dem Wasser kontinuierlich aus einem wasserhaltigen Gemisch von Methyl-tert-butylether und C₄-Kohlenwasserstoffen bei der Auftrennung des Gemisches in einer Destillationskolonne abgetrennt wird.

Diese Aufgabe wird erfindungsgemäß dadurch gelöst, daß das Gemisch auf die Destillationskolonne aufgegeben, C₄-Kohlenwasserstoffe und Wasser azeotrop abdestilliert, das Destillat kondensiert und in eine wasserreiche Phase und eine C₄-Kohlenstoff-reiche Phase aufgetrennt und ein Teil der C₄-Kohlenstoff-reichen Phase als Rückfluß auf den Kolonnenkopf zurückgegeben und eine zweite wasserreiche Phase unterhalb der Gemischzuführung in der Destillationskolonne abgeschieden und seitlich aus der Destillationskolonne abgezogen wird.

Hierbei setzen die eine oder mehrere überkopf siedenden Substanzen die Lösung von Wasser in den zu reinigenden organischen Substanzen oder Substanzgemischen herab oder bilden mit Wasser nur unzureichende Azeotrope und bewirken si ein Überschreiten der Lösungsgrenze für Wasser in den organischen Substanzen oder Substanzgemischen.

Das Verfahren bezieht sich auf zu reinigende organische Substanzen oder Substanzgemische, die Wasser nur begrenzt aufzunehmen vermögen. Das Wasser wird, bedingt durch die Anwesenheit einer oder meherer Substanzen, die die Lösung des Wassers in den zu reinigenden organischen Substanzen oder den Substanzgemischen herabsetzen, ausgeschieden.

Solche Lösungsbegrenzer sind zunächst die rein darzustellenden Produkte, wenn durch Überschreiten der Lösungsgrenze für Wasser dieses zum Ausscheiden gezwungen wird. Das gilt z.B. für die Lösungsgrenze für Wasser in in MTBE. Hierbei wird die Lösungsgrenze für Wasser in dem zu erzeugenden Produkt dadurch überschritten, daß durch die gleichzeitig im Destillationseinsatz enthaltenen leichtsiedenden Produkte der Wasseraustrag in das Kopfprodukt verhindert oder begrenzt wird.

Das erfindungsgemäße Verfahren beruht darauf, daß in der Kolonne ein Zustand einer dauernden Wassersättigung eingestellt wird, so daß das mit dem Destillationseinsatz kontinuierlich eingetragene Wasser sich wegen Übersättigung ausscheiden muß und unverdampft abgezogen werden kann.

Stets ist nach der Wasserabscheidung in der Kolonne der Restwassergehalt in dem ablaufenden flüssigen Kolonnenprodukt geringer als der mögliche Wassergehalt in dem aufsteigenden dampfförmigen Kolonnenprodukt, so daß zwangsläufig ein trockenes Produkt am Kolonnensumpf gewährleistet ist.

Überraschenderweise wurde somit gefunden, daß bei Umkehrung des sonst angewandten Trocknungsprinzips: Verdampfung mit Azeotropbildung und Kondensation mit Phasenzerfall und Wasserabscheidung, bessere Resultate erzielt werden.

Hierzu wird erfindungsgemäß bereits in der Destillationskolonne, und zwar immer unterhalb der Produktzuführung, die an dieser Stelle für die Wasserunlöslichkeit günstige Produktzusammensetzung zur Abscheidung sowie Abtrennung von Wasser genutzt. Damit kann auf die Bildung von Wasser-Azeotropen überkopf der Kolonne verzichtet bzw. die Menge von Wasser-Azeotropen überkopf der Kolonne herabgesetzt werden.

Der aus dem Lösungsgleichgewicht an dieser Wasserabscheidestelle resultierende Restwassergehalt in dem in den Kolonnenabtriebsteil ablaufenden flüssigen Produktstrom wird durch die im Abtriebsteil aufsteigenden, durch Verdampfung erzeugten organischen Brüden azeotrop wieder in den Kolonnenteil oberhalb der Wasserabscheidung zurückgeführt. Für diesen Wasserrücktransport auf der Basis der jeweils möglichen azeotropen Zusammensetzungen ist ein Verdampfungsaufwand erforderlich, der meistens durch die überlagerte Trennaufgabe zur Abtrennung von anderen Verunreinigungen gegeben ist. Damit ist für die Abtrennung von Wasser kein zusätzlicher Verdampfungsaufwand erforderlich.

In den Fällen, wo als Trennaufgabe nur die Abtrennung von Wasser gefordert wird, ist der Verdampfungsaufwand nur auf den Wassereintrag und auf die azeotropen Eigenschaften im Abtriebsteil der Kolonne durch dementsprechenden Zwangsrücklauf auf die Kolonne einzustellen.

Darüberhinaus kann auch durch gezielten und kontrollierten Wasserabzug am Abscheideboden eine für bestimmte Trennaufgaben gewünschte Wasserkonzentration im Abtriebsteil der Kolonne eingestellt und eingehalten werden.

Es ist so möglich, das aus den wasserhaltigen organischen Gemischen abzutrennende Wasser ganz oder teilweise ohne zusätzlichen oder mit stark verringertem Verdampfungsaufwand an einem unter dem Einspeisepunkt vorhandenen Wasserabscheider in der Destillationskolonne abzuziehen.

Technisch kann das erfinderische Prinzip dadurch realisiert werden, daß eine insbesonders für die azeotrope Trocknung geeignete Destillationskolonne durch einen eingebauten Wasserabscheider ergänzt wird. Der Wasserabscheider sollte dort, wo die höchste Wasserabscheidung zu erwarten ist, auf jeden Fall nicht höher als der Einspeiseboden oder kurz darunter, installiert werden.

Es ist dabei unbedeutend, ob als technische Lösung ein externer Wasserabscheider außerhalb der Kolonne oder als Kaminbodenwasserabscheider innerhalb der Kolonne realisiert wird. Wichtig ist, daß das gesamte, in den Abtriebsteil der Destillationskolonne ablaufende Produkt über den Wasserabscheider geführt wird. Durch diese erfindungsgemäße Voraussetzung ist gewährleistet, daß unabhängig von der Wasserkonzentration im Einsatzprodukt der Wassereintrag in den Abtriebsteil der Kolonne immer durch das Löslichkeitsprodukt am Wasserabscheideboden einzustellen ist.

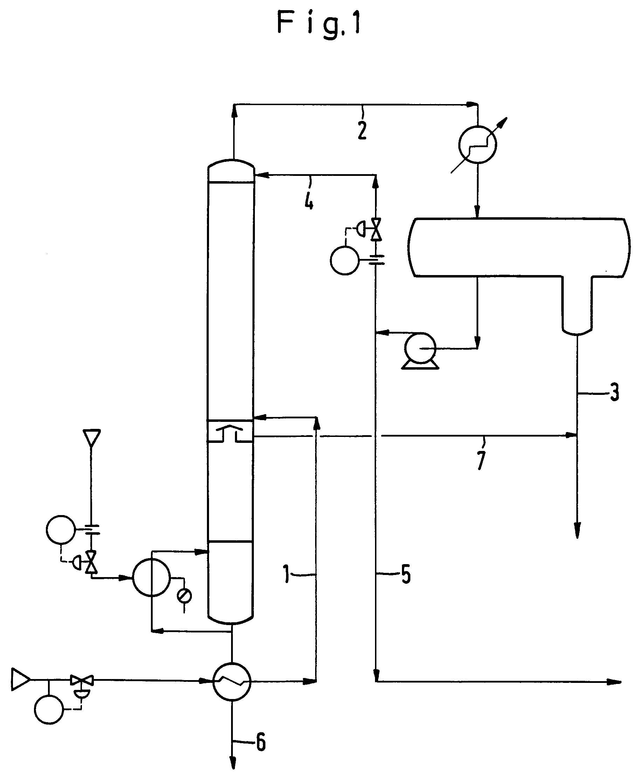

Das nachfolgende Beispiel erläutert die Erfindung anhand der Figur 1. Das Beispiel zeiget, daß durch das erfindungsgemäße Verfahren eine erhebliche Reduzierung des Destillationsaufwandes für die Abtrennung von Wasser erreicht oder aber ein zusätzlicher, über die eigentliche Trennaufgabe hinausgehender Destillationsaufwand für die Abtrennung von Wasser vermieden wird.

### Beispiel

In einer in Figur 1 schematisch dargestellten, kontinuierlich zu betreibenden Destillationskolonne wurde ein wasserhaltiges Gemisch aus Methyl-tert-butylether (MTBE) und C₄-Kohlenwasserstoffen im Verhältnis von 65 : 35 Gewichtsteilen bei einem Druck von 6 bar destillativ getrennt. Die Produktaufgabe erfolgte über Leitung 1 im unteren Kolonnenteil, 15 Böden oberhalb des Kolonnensumpfes.

Mit den C₄-Kohlenwasserstoffen als Kopfprodukt wurde in die Kolonne eingebrachtes Wasser über Leitung 2 azeotrop ausgetragen, nach der Kondensation des Kopfproduktes abgeschieden und aus dem Rücklaufbehälter über Leitung 3 abgezogen.
Zur Aufrechterhaltung der Trennung MTBE/C₄-Kohlenwasserstoffe wurde ein Teil des Kopfproduktes über Leitung 4 wieder als Rücklauf auf die Kolonne zurückgeführt. Der im Einsatzprodukt enthaltene Teil des Kopfproduktes wurde über Leitung 5 als Destillat abgezogen.
Aus dem Verhältnis von Rücklauf (R) zu Destillat (D) errechnet sich das Rücklaufverhältnis R/D, das maßgeblich den Energieverbrauch bei dieser Destillation bestimmt.

In den Vergleichsversuchen 1.1 und 1.2 wird gezeigt, daß dieses Rücklaufverhältnis bei der gleichzeitigen Trocknung von wasserhaltigen Einsatzmischungen in ungünstigen Fällen höher anzusetzen ist,als es für die Trennung von MTBE und C₄-Kohlenwasserstoffen erforderlich wäre.

### Versuch 1.1 (Vergleich)

Bei einem Wassereintrag von 9,1 g in 1260 g Einsatzgemisch = 0,72 Gew.% Wasser wurden durch eine Rücklaufmenge von 860 g und ein Rücklaufverhältnis von R/D = 2,0 1280 g C₄-Kopfprodukt mit 0,70 Gew. % Wasser aus der Kolonne abdestilliert. Aus diesem Kopfprodukt konnten 9,0 g Wasser als flüssige Phase abgeschieden und abgezogen werden. Am Kolonnensumpf fielen über Leitung 6 830 g C₄-freier MTBE mit einem Wassergehalt von kleiner 0,03 Gew.% an.

### Versuch 1.2 (Vergleich)

Bei einem Wassereintrag von 8,5 g in 1260 g Einsatzgemisch = 0,67 Gew.% Wasser wurden durch eine Rücklaufmenge von 540 g und ein Rücklaufverhältnis von R/D = 1,2 970 g C₄-Kopfprodukt mit 0,57 Gew.% Wasser abdestilliert. Aus diesem Kopfprodukt konnten 5,5 g Wasser als flüssige Phase abgeschieden und abgezogen werden. Durch das unzureichende Schleppvermögen des Kopfproduktes für Wasser wurden am Kolonnensumpf über Leitung 6 830 g C₄-freier MTBE mit einem Wassergehalt von 0,7 Gew.% erhalten.

Dagegen können erfindungsgemäß durch Einbau eines Wasserabscheiders an einer geeigneten Stelle der Kolonne unterhalb des Aufgabebodens und Abzug von Wasser über Leitung 7 folgende Ergebnisse erzielt werden:

### Versuch 1.3 (erfindungsgemäß)

Bei einem Wassereintrag von 9,8 g in 1400 g Einsatzprodukt = 0,70 Gew.% Wasser wurden durch eine Rücklaufmenge von 250 g und ein Rücklaufverhältnis von R/D = 0,5 740 g C₄-Kopfprodukt mit 0,51 Gew.% Wasser abdestilliert. Aus diesem Kopfprodukt konnten 3,6 g Wasser als flüssige Phase abgeschieden und abgetrennt werden. Am Wasserabscheider in der Kolonne konnten über Leitung 7 6,1 g Wasser abgezogen werden. Am Kolonnensumpf fielen über Leitung 6 910 g C₄-freier MTBE mit einem Wassergehalt von kleiner 0,02 Gew.% an.

### Versuch 1.4 (erfindungsgemäß)

Bei einem Wassereintrag von 35 g in 1400 g Einsatzprodukt = 2,4 Gew.% Wasser wurden durch eine Rücklaufmenge von 250 g und ein Rücklaufverhältnis von R/D = 0,5 740 g C₄-Kopfprodukt mit 0,5 Gew.% Wasser abdestilliert. Aus diesem Kopfprodukt konnten 3,7 g Wasser als flüssige Phase abgeschieden und abgetrennt werden. Am Wasserabscheider in der Kolonne wurden über Leitung 7 31,3 g Wasser abgezogen. Am Kolonnensumpf fielen über Leitung 6 910 g C₄-freier MTBE mit einem Wassergehalt von kleiner 0,02 Gew.% an.

## Patentansprüche

1. Verfahren zur kontinuierlichen Abtrennung von Wasser aus einem wasserhaltigem Gemisch von Methyl-tert-butylether und C₄-Kohlenwasserstoffen bei der Auftrennung des Gemisches in einer Destillationskolonne, **dadurch gekennzeichnet**,
daß das Gemisch auf die Destillationskolonne aufgegeben, C₄-Kohlenwasserstoffe und Wasser azeotrop abdestilliert, das Destillat kondensiert und in eine wasserreiche Phase und eine C₄-Kohlenstoff-reiche Phase aufgetrennt und ein Teil der C₄-Kohlenwasserstoff-reichen Phase als Rückfluß auf den Kolonnenkopf zurückgegeben und eine zweite wasserreiche Phase unterhalb der Gemischzuführung in der Destillationskolonne abgeschieden und seitlich aus der Destillationskolonne abgezogen wird.

## Claims

1. A process for the continuous separation of water from a water-containing mixture of methyl-tert-butyl ether and C₄-hydrocarbons during the splitting-up of the mixture in a distillation column, said process comprising feeding the mixture to the top of the distillation column, removing C₄-hydrocarbons and water by azeotropic distillation, condensing the distillate, splitting the same into a water-rich phase and a C₄-hydrocarbons-rich phase, recycling part of the C₄-hydrocarbons-rich phase to the top of the column, separating the second water-rich phase in the distillation column below the feed point for the mixture and removing the same at the side of the distillation column.

## Revendications

1. Procédé de séparation continue d'eau d'un mélange aqueux d'éther de méthyle et de tertio-butyle et d'hydrocarbures en C₄ lors du fractionnement du mélange dans une colonne de distillation, caractérisé en ce qu'on charge le mélange sur la colonne de distillation, on sépare les hydrocarbures en C₄ et l'eau par distillation azéotropique, on condense le distillat et on le divise en une phase riche en eau et une phase riche en hydrocarbures en C₄ et on recycle une partie de la phase riche en hydrocarbures en C₄ comme reflux à la tête de la colonne, on sépare une seconde phase riche en eau au-dessous de l'arrivée du mélange dans la colonne de distillation et on la fait sortir latéralement de la colonne de distillation.
